(19) Europäisches Patentamt
European Patent Office
Office européen des brevets

(11) Publication number: 0 084 720
B1

(12) **EUROPEAN PATENT SPECIFICATION**

(45) Date of publication of patent specification: **07.08.85**

(51) Int. Cl.$^4$: **C 07 D 237/18,** A 61 K 31/50

(21) Application number: **82306742.6**

(22) Date of filing: **17.12.82**

(54) **(3,6-Pyridazinediylbis(thio))bis-(2,2-dimethylalkanoic acids) and derivatives thereof, suitable as anti-arteriosclerotic agents, processes for the production of such compounds and pharmaceutical compositions including such compounds.**

(30) Priority: **18.12.81 US 332058**

(43) Date of publication of application:
**03.08.83 Bulletin 83/31**

(45) Publication of the grant of the patent:
**07.08.85 Bulletin 85/32**

(84) Designated Contracting States:
**AT BE CH DE FR GB IT LI LU NL SE**

(56) References cited:
**DE-B-1 098 000**
**DE-B-1 099 544**
**US-A-4 058 520**

(73) Proprietor: **WARNER-LAMBERT COMPANY**
**201 Tabor Road**
**Morris Plains New Jersey 07950 (US)**

(72) Inventor: **Sircar, Ila**
**3615 Charter Place**
**Ann Arbor Michigan 48105 (US)**

(74) Representative: **Jones, Michael Raymond et al**
**HASELTINE LAKE & CO. Hazlitt House 28**
**Southampton Buildings Chancery Lane**
**London WC2A 1AT (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 084 720 B1

**Description**

This invention relates to [3,6-pyridazinediylbis(thio)]bis-[2,2-dimethylalkanoic acids] and derivatives thereof, suitable as anti-arteriosclerotic agents; processes for the production of such compounds; and pharmaceutical compositions including such compounds.

Elevated levels of blood cholesterol and blood lipids are conditions which are believed to be related to the onset of arteriosclerosis. Thus, compounds capable of reducing the levels of these blood constituents are recognised as potentially useful anti-arteriosclerotic agents.

The compounds of the present invention are useful as anti-arteriosclerotic agents and are capable of elevating the high density lipoprotein fraction of cholesterol (HDL-cholesterol), which effect is known to lower the risk factor of coronary heart disease (Gordon, T. et al., High Density Lipoprotein as a Protective Factor Against Coronary Heart Disease, May 1977, The American Journal of Medicine, Vol. 62, pp. 707—714). Certain compounds of the present invention also are able to reduce the low density lipoprotein fraction of cholesterol (LDL-cholesterol), thus further reducing the risk factor of coronary heart disease.

One aspect of the present invention provides compounds having the following structural formula I:

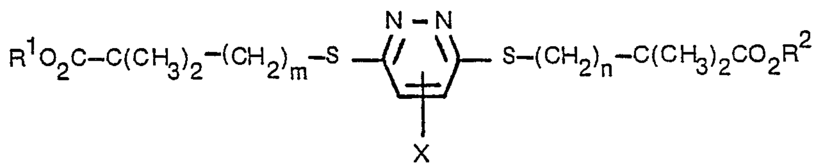

I

wherein each of m and n, which are the same or different, is an integer from 3 to 6; X is a hydrogen atom, a phenyl radical or an alkyl radical of from 1 to 6 carbon atoms; each of $R^1$ and $R^2$, which are the same or different, is a hydrogen atom, an alkyl radical of from 1 to 6 carbon atoms, or a pharmaceutically acceptable cation; and pharmaceutically acceptable salts thereof.

Conveniently, but not necessarily, $R^1$ and $R^2$ are the same; and m and n are the same.

Preferably m and n are each 3.

Another aspect of the present invention provides a process for producing the compounds of the first-mentioned aspect, which process comprises reacting a pyridazine having the following structural formula II:

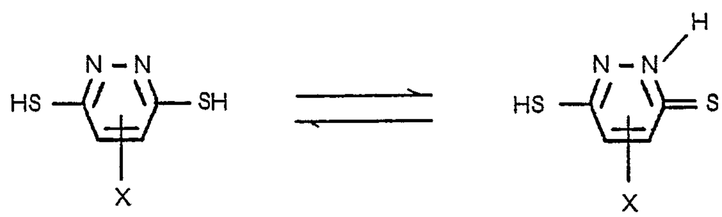

II

with one or both of the 2,2-dimethylalkanoic acid derivatives having the following structural formulae III:

$$Y^1—(CH_2)_m—C(CH_3)_2—CO_2R^1$$

$$Y^2—(CH_2)_n—C(CH_3)_2—CO_2R^2 \qquad III$$

wherein m, n, $R^1$, $R^2$ and X are as defined above and each of $Y^1$ and $Y^2$, which are the same or different, is a halogen atom, there being sufficient of the 2,2-dimethylalkanoic acid derivative(s) for two molecules of the acid derivative to react with one of the pyridazine; and, where appropriate, converting the product of the reaction to a pharmaceutically acceptable acid addition salt.

A further aspect of the present invention provides an alternative process for producing compounds of the first-mentioned aspect, which process comprises reacting a pyridazine having the following structural formula:

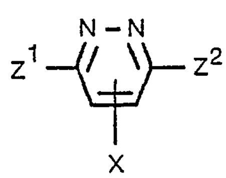

with one or both of the 2,2-dimethylalkanoic acid derivatives having the following structural formulae:

$$HS(CH_2)_m—C(CH_3)_2—CO_2R^1$$

$$HS(CH_2)_n—C(CH_3)_2—CO_2R^2$$

wherein m, n, $R^1$, $R^2$ and X are as defined above, and each of $Z^1$ and $Z^2$, which may be the same or different, is a halogen atom, there being sufficient of the 2,2-dimethylalkanoic acid derivative(s) for two molecules of the acid derivative to react with one molecule of the pyridazine; and, where appropriate, converting the product of the reaction to a pharmaceutically acceptable acid addition salt.

In the process of the second and third-mentioned aspects of the invention, conveniently $Y^1$ and $Y^2$ are the same, and $Z^1$ and $Z^2$ are the same, respectively. Also preferably there are two equivalents of the 2,2-dimethylalkanoic acid derivative per equivalent of pyridazine.

The present invention also provides a pharmaceutical composition useful for treating arteriosclerosis in a mammal, comprising a compound having the structural formula I or mixture thereof, in combination with a pharmaceutically acceptable carrier.

The compounds of the present invention may be prepared by any of several processes which are to be considered as equivalent for purposes of the present invention.

One such process involves the reaction between a pyridazine having the structural formula II,

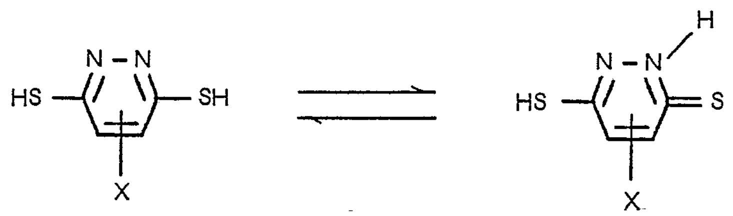

II

and two equivalents of a 2,2-dimethylalkanoic acid derivative having the structural formula III

$$Y^2—(CH_2)_n—C(CH_3)_2—CO_2R^2 \quad (III)$$

wherein $R^2$, n and X are defined above, and $Y^2$ is a halogen.

This reaction is most conveniently carried out in solution in a non-reactive solvent such as dimethoxyethane, dioxane, tetrahydrofuran, acetone, acetonitrile, dimethylsulphoxide or dimethylformamide at a temperature of up to about 120°C in the presence of an acid acceptor such as a tertiary amine, pyridine or an alkali metal or alkaline earth metal carbonate or bicarbonate. In a preferred process, Y represents bromine and the above reaction is carried out in dimethylformamide solution at a temperature of 60—70°C in the presence of potassium carbonate. In this preferred procedure, the reaction is usually substantially complete in from 6 to 8 hours. The carboxylic acid esters and acids of structural formula III may be prepared as described in United States Patent 3,674,836 or by obvious variations thereof.

In a second process for producing the compounds of the present invention, a pyridazine of the structural formula IV

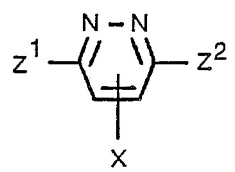

IV

is reacted with 2 equivalents of a 2,2-dimethylalkanoic acid derivative having the structural formula V

$$HS(CH_2)_nC(CH_3)_2CO_2R^2$$

wherein $R^2$, n and X are defined above, and each of $Z^1$ and $Z^2$ is a halogen.

The reaction between Compounds IV and V is preferably carried out in a non-reactive solvent such as toluene or xylene in the presence of a strong base such as sodium hydride.

In a preferred procedure, compound V is first reacted with sodium hydride in toluene without the addition of external heating. Subsequently, a solution of IV in toluene is slowly added and the mixture is allowed to reflux overnight. The product is isolated and purified by standard procedures. In this preferred procedure, substituents $Z^1$ and $Z^2$ represent chlorine. The compounds of formula IV may be prepared as described in Bull. Soc. Chim., 1004(1957).

The compounds of the invention of formula I wherein each of $R^1$ and $R^2$ is hydrogen form pharmaceutically acceptable salts with both organic and inorganic acids and bases. Examples of suitable acids for salt formation are hydrochloric, sulphuric, phosphoric, acetic, citric, oxalic, malonic, salicyclic, malic, fumaric, succinic, ascorbic, maleic and methane-sulphonic. The salts are prepared by contacting the free

base form with an equivalent amount of the desired acid in the conventional manner. The free base forms may be regenerated by treating the salt form with a base. For example, dilute aqueous base solutions may be utilized. Dilute aqueous sodium hydroxide, potassium carbonate, ammonia and sodium bicarbonate solutions are suitable for this purpose. The free base forms differ from their respective salt forms somewhat in certain physical properties such as solubility in polar solvents, but the salts are otherwise equivalent to their respective free base forms for the medicinal purposes of the present invention.

Examples of suitable bases for salt formation are sodium hydroxide, sodium carbonate, sodium bicarbonate, potassium hydroxide, calcium hydroxide, ammonia and organic amines. The salts are prepared by contacting the free acid form with an equivalent amount of the desired base in a conventional manner. The free acid forms may be regenerated by treating the salt form with an acid. For example, dilute aqueous acid solutions may be utilized. Dilute aqueous hydrochloric acid, sulphuric or acetic acid are suitable for this purpose. The free acid forms differ from their respective salt forms somewhat in certain physical properties such as soublility in polar solvents, but the salts are otherwise equivalent to their respective free acid forms for the medicinal purposes of the present invention.

The compounds of the present invention wherein each of $R^1$ and $R^2$ is alkyl of from 1 to 6 carbon atoms form pharmaceutically acceptable salts with both organic and inorganic acids. Examples of suitable acids and methods of preparation of the salts are identical to those given above.

The compounds of the present invention can exist in unsolvated as well as solvated forms, including hydrated forms. In general, the solvated forms, with pharmaceutically acceptable solvents such as water, ethanol and the like are equivalent to the unsolvated forms for the medicinal purposes of the present invention.

The term "halogen" as used herein is intended to include chlorine, bromine and iodine.

The alkyl groups contemplated by the present invention, unless specified otherwise, comprise both straight and branched carbon chains of from 1 to 6 carbon atoms. Representative of such groups are methyl, ethyl, isopropyl, butyl (i.e. n-butyl), pentyl (i.e. n-pentyl), and 3-methylpentyl.

The compounds of the present invention are new chemical substances of value as pharmacological agents for the treatment of arteriosclerosis in warm-blooded animals. The anti-arteriosclerotic activity of representative compounds of the invention was established by the Screening procedure described in Maxwell, R.E., Nawrocki, J.W., and Uhlen-dorf, P.D., Artery, *1*, 303 (1978). This procedure is incorporated by reference herein. Utilizing this procedure at a dose level of 50 mg/kg, the results shown in the following Table were obtained for representative compounds of the present invention. A compound is considered active if it increases the HDL cholesterol fraction by 50%

TABLE

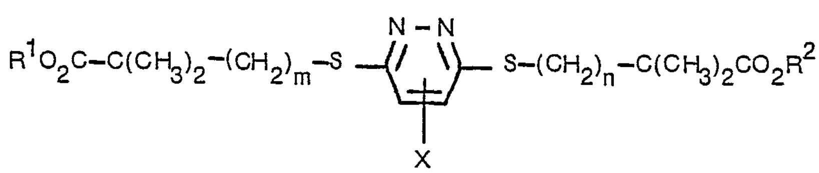

| $R^1 = R^2$ | X | m = n | % Change in Cholesterol | % Change Triglyceride | % Change in HDL | % Change in LDL | % Change in Liver wt. |
|---|---|---|---|---|---|---|---|
| $CH_3$ | H | 3 | −24 | +166 | +1129 | −67 | +32 |
| $CH_3$ | $C_6H_5$ | 3 | −43 | 0 | + 64 | −51 | 0 |
| $CH_3$ | $CH_3$ | 3 | 0 | + 34 | + 348 | −42 | +26 |
| $CH_3$ | H | 4 | −14 | + 15 | + 134 | −25 | + 7 |
| $CH_3$ | H | 6 | 0 | − 26 | + 33 | −17 | + 8 |

An increase in liver weight is indicative of hepatomegaly and hepatic peroxisome proliferation. Both are undesirable side effects of the known anti-artereosclerotic agents, Reddy, J.F., and Krishnakantha, T.P., Science, *190*, 787 (1975).

The compounds of the present invention can be prepared and administered in a wide variety of oral and parenteral dosage forms. It will be clear to those skilled in the art that the following dosage forms may comprise, as the active component, either a compound of formula I, or a corresponding pharmaceutically acceptable salt of a compound of formula I, or a mixture of such compounds and/or salts.

For preparing pharmaceutical compositions from the compounds described in the present invention, inert, pharmaceutically acceptable carriers can be either solid or liquid. Solid form preparations include powders, tablets, dipersible granules, capsules, cachets and suppositories. A solid carrier can be one or more substances which may also act as a diluent, flavouring agent, solubilizer, lubricant, suspending agent, binder or tablet-disintegrating agent; it can also be an encapsulating material. In powders, the carrier is a finely divided solid which is admixture with the finely divided active compound. In the tablet the active compound is mixed with a carrier having the necessary binding properties in suitable proportions and compacted in the shape and size desired. The powders and tablets preferably contain from 5 to 70, more usually from 10 to 70, percent of the active ingredient. Suitable solid carriers are magnesium carbonate, magnesium stearate, talc, sugar, lactose, pectin, dextrin, starch, gelatin, tragacanth, methyl cellulose, sodium carboxymethyl cellulose, a low melting wax and cocoa butter. The term "preparation" is intended to include the formulation of the active compound with encapsulating material as carrier providing a capsule in which the active component (with or without other carriers) is surrounded by carrier, which is thus in association with it, Similarly, cachets are included. Tablets, powders, cachets and capsules can be used as solid dosage forms suitable for oral administration.

Liquid form preparations include solutions, suspensions and emulsions. As an example there may be mentioned water or water-propylene glycol solutions for parenteral injection. Liquid preparations can also be formulated in solution in aqueous polyethylene glycol solution. Aqueous solutions suitable for oral use can be prepared by dissolving the active component in water and adding suitable colourants, flavours, stabilizing and thickening agents as desired. Aqueous suspensions suitable for oral use can be made by dispersing the finely divided active component in water with viscous material, e.g., natural or synthetic gum, resin, methyl cellulose, sodium carboxymethyl cellulose or another well-known suspending agent.

Preferably, the pharmaceutical preparation is in unit dosage form. In such form, the preparation is subdivided into unit doses containing appropriate quantities of the active component. The unit dosage form can be a packaged preparation, the package containing discrete quantities of preparation, for example, packeted tablets, capsules and powders in vials or ampoules. The unit dosage form can also be a capsule, cachet or tablet itself or it can be the appropriate number of any of these packaged forms.

The quantity of active compound in a unit dose of preparation may be varied or adjusted from 1 mg to 100 mg according to the particular application and the potency of the particular active ingredient.

In therapeutic use as agents for treating aterioslcerosis, the compounds of the present invention may be administered at the initial dosage of from 10 mg to 500 mg per kilogram of body weight daily. A daily dose range of from 10 mg to 250 mg per kilogram is preferred. The dosages, however, may be varied depending upon the requirements of the patient, the severity of the condition being treated and the compound being employed. Determination of the proper dosage for a particular situation is within the skill of the art. Generally, treatment is initiated with smaller dosages which are less than the optimum dose of the comound. Thereafter, the dosage is increased by small increments until the optimum effect under the circumstances is reached. For convenience, the total daily dosage may be divided and administered in portions during the day if desired.

The following non-limiting examples illustrate the preferred processes for producing some of the comopunds of the present invention.

Example 1

A mixture of 3-mercapto-6-(1H)-pyridazinethione (14.2 g, 0.1 mole) prepared according to A. Pollak, et. al., Canadian Journal of Chemistry, *44*, 829 (1966), anhydrous $K_2CO_3$ (0.2 mole, 27.0 g) and methyl-5-bromo-2,2-dimethylpentanoate (0.23 mole, 56 ml) in dimethyl-formamide (300 ml) was stirred at 75—80°C for 13—14 hours. The mixture was cooled, filtered from the inorganic salts, and dimethylformamide was distilled off under reduced pressure. The residue was distilled under vacuum to remove the excess bromo-ester and then chromatographed over silica gel. The product was eluted out with a 1:1 mixture of hexane and diisopropyl ether and then purified by crystallization from hexane to yield 26.0 g of the product, dimethyl-5,5'-[3,6-pyridazinediylbis(thio)]bis[2,2-dimethyl-pentanoate] m.p. 32—33°C.

Following the general procedure of Example 1, but with the substituted pyridazines, the following additional products were obtained.

Example 2

From 4-phenyl-3,6-dimercaptopyridazine, the product dimethyl-5,5'-[(4-phenyl-3,6-pyridazinediyl)bis(thio)]bis[2,2-dimethylpentanoate] was obtained.

Example 3

From 4-methyl-3,6-dimercaptopyridazine, (J. Druey, K.D. Meier and K. Eichenberger; Helv. Chim. Acta. *37*, 121, 1954) the product dimethyl-5,5'-[(4-methyl-3,6-pyridazinediyl)bis(thio)]bis[2,2-dimethyl-pentanoate] was obtained.

Example 4

Following the procedure of Example 1, with the substitution of methyl 6-bromo-2,2-dimethylhexanoate in place of methyl 5-bromo-2,2-dimethylpentanoate, the product dimethyl 6,6'-[3,6-pyridazinediylbis(thio)]-bis[2,2-dimethylhexanoate] was obtained.

Example 5

A solution of 1.07 g dimethyl 5,5'-[3,6-pyridazinediylbis(thio)]bis[2,2-dimethylpentanoate] in methanol (40 ml) was heated to reflux with a solution of NaOH (0.22 g) in water (10 ml) for 4 hours. Methanol was distilled off and the solution was acidified. Upon usual work up, the product obtained was 5,5'-[3,6-pyridazinediylbis(thio)]bis[2,2-dimethylpentanoic acid], mp 112—114°C following crystallization from chloroform/petroleum ether.

Preparative Example

3-Mercapto-4-phenyl-6(1H)-pyridazinethione

A mixture of phosphorus pentasulphide (15.0 g) and 4-phenyl-3,6-pyridazinedione (2.5 g) in pyridine (38 ml) was refluxed for 3 hours. Pyridine was distilled off and the residue was poured into water and the mixture was heated to boiling until complete dissolution took place. The solution was then made alkaline with sodium hydroxide followed by acidification. The solids were filtered, washed with water, and dried. The product obtained had mp. 146—148°C, followed by crystallization from toluene.

**Claims for the Contracting States: BE CH DE FR GB IT LI LU NL SE**

1. A compound having the following structural formula

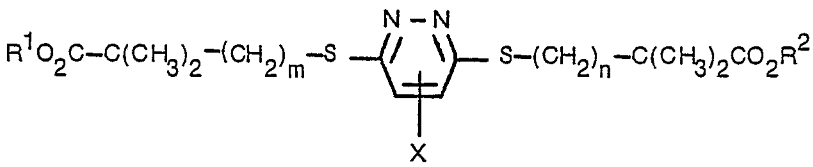

wherein each of m and n, which are the same or different, is an integer from 3 to 6; X is a hydrogen atom, a phenyl radical or an alkyl radical of from 1 to 6 carbon atoms; each of $R^1$ and $R^2$, which are the same or different, is a hydrogen atom, an alkyl radical of from 1 to 6 carbon atoms, or a pharmaceutically acceptable cation; or a pharmaceutically acceptable acid addition salt thereof.

2. A compound as claimed in Claim 1, wherein m and n are the same, and $R^1$ and $R^2$ are the same.

3. A compound as claimed in Claim 2, wherein each of m and n is 3.

4. A compound as claimed in Claim 2 or 3, wherein each of $R^1$ and $R^2$ is a methyl radical.

5. A compound as claimed in Claim 2 or 3, wherein each of $R^1$ and $R^2$ is a hydrogen atom.

6. The compound dimethyl 5,5'-[3,6-pyridazinediylbis(thio)]bis [2,2-dimethylpentanoate]; dimethyl 5,5'-[(4-phenyl-3,6-pyridazinediyl)bis(thio)]bis[2,2-dimethylpentanoate]; or dimethyl 5,5'-[(4-methyl-3,6-pyridazinediyl)bis(thio)]-bis[2,2-dimethylpentanoate]; or a pharmaceutically acceptable acid addition salt thereof.

7. The compound dimethyl 6,6'-[3,6-pyridazinediylbis(thio)]bis(2,2-dimethylhexanoate] or a pharmaceutically acceptable acid addition salt thereof.

8. The compound dimethyl 8,8'-[3,6-pyridazinediylbis(thio)]bis[2,2-dimethyloctanoate] or a pharmaceutically acceptable acid addition salt thereof.

9. The compound 5,5'-[3,6-pyridazinediylbis(thio)]bis[2,2-dimethylpentanoic acid] or a pharmaceutically acceptable acid and/or base addition salt(s) thereof.

10. A process for producing a chemical compound as claimed in Claim 1, which comprises reacting a pyridazine having the following structural formula

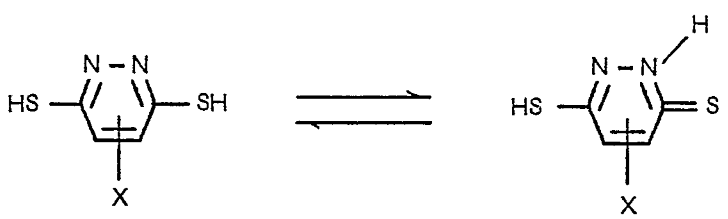

with one or both of the 2,2-dimethylalkanoic acid derivatives having the following structural formulae

$$Y^1—(CH_2)_m—C(CH_3)_2—CO_2R^1$$

$$Y^2—(CH_2)_n—C(CH_3)_2—CO_2R^2$$

wherein m, n, $R^1$ and X are as defined in Claim 1 and each of $Y^1$ and $Y^2$, which are the same or different, is a halogen atom, there being sufficient of the 2,2-dimethylalkanoic acid derivative(s) for two molecules of the acid derivative to react with one of the pyridazine; and, where appropriate, converting the product of the reaction to a pharmaceutically acceptable acid addition salt.

11. A process for producing a chemical compound as claimed in Claim 1, which comprises reacting a pyridazine having the following structural formula

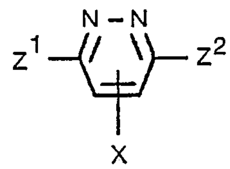

with one or both of the 2,2-dimethylalkanoic acid derivatives having the following structural formulae

$$HS(CH_2)_m—C(CH_3)_2—CO_2R^1$$

$$HS(CH_2)_n—C(CH_3)_2—CO_2R^2$$

wherein m, n, $R^1$, $R^2$ and X are as defined in Claim 1 and each of $Z^1$ and $Z^2$, which may be the same or different, is a halogen atom, there being sufficient of the 2,2-dimethylalkanoic acid derivative(s) for two molecules of the acid derivative to react with one molecule of the pyridazine; and, where appropriate, converting the product of the reaction to a pharmaceutically acceptable acid addition salt.

12. A composition useful for treating arteriosclerosis in a mammal, which comprises a compound as claimed in any one of Claims 1 to 9, or a mixture thereof, in combination with a pharmaceutically acceptable carrier.

**Claims for the Contracting State: AT**

1. A process for producing a compound having the following structural formula

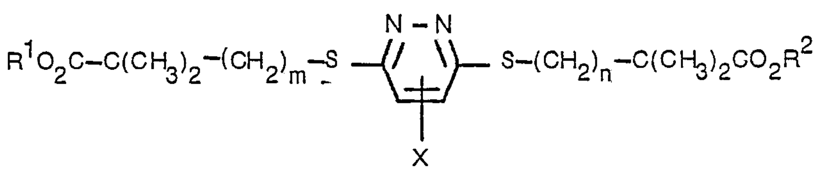

wherein each of m and n, which are the same or different, is an integer from 3 to 6; X is a hydrogen atom, a phenyl radical or an alkyl radical of from 1 to 6 carbon atoms; each of $R^1$ and $R^2$, which are the same or different, is a hydrogen atom, an alkyl radical of from 1 to 6 carbon atoms, or a pharmaceutically acceptable acid addition salt thereof: which process comprises reacting a pyridazine having the following structural formula II:

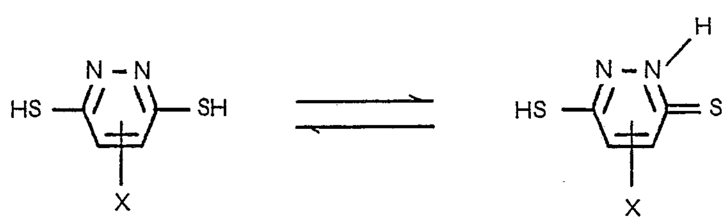

II

with one or both of the 2,2-dimethylalkanoic acid derivatives having the following structural formulae III:

$$Y^1—(CH_2)_m—C(CH_3)_2—CO_2R^1$$

$$Y^2—(CH_2)_n—C(CH_3)_2—CO_2R^2$$

III

wherein m, n, $R^1$ and X are as defined in Claim 1 and each of $Y^1$ and $Y^2$, which are the same or different, is a halogen atom, there being sufficient of the 2,2-dimethylalkanoic acid derivative(s) for two molecules of the acid derivative to react with one of the pyridazine; and, where appropriate, converting the product of the reaction to a pharmaceutically acceptable acid addition salt.

2. A process for producing a compound having the following structural formula

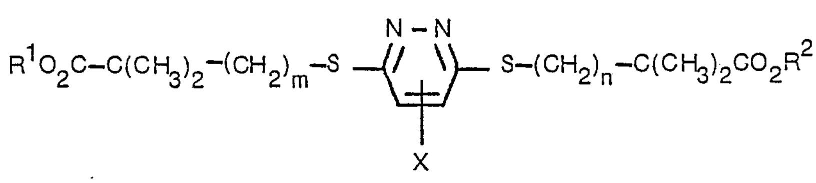

wherein each of m and n, which are the same or different, is an integer from 3 to 6; X is hydrogen atom, a phenyl radical or an alkyl radical of from 1 to 6 carbon atoms; each of $R^1$ and $R^2$, which are the same or different, is a hydrogen atom, an alkyl radical of from 1 to 6 carbon atoms, or a pharmaceutically acceptable cation; or a pharmaceutically acceptable acid addition salt thereof: which comprises reacting a pyridazine having the following structural formula

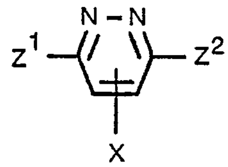

with one or both of the 2,2-dimethylalkanoic acid derivatives having the following structural formulae

$$HS(CH_2)_m—C(CH_3)_2—CO_2R^1$$

$$HS(CH_2)_n—C(CH_3)_2—CO_2R^2$$

wherein m, n, $R^1$, $R^2$ and X are as defined in Claim 1 and each of $Z^1$ and $Z^2$, which may be the same or different, is a halogen atom, there being sufficient of the 2,2-dimethylalkanoic acid derivative(s) for two molecules of the acid derivative to react with one molecule of the pyridazine; and, where appropriate, converting the product of the reaction to a pharmaceutically acceptable acid addition salt.

3. A process as claimed in Claim 1 or 2, wherein m and n are the same, and $R^1$ and $R^2$ are the same.

4. A process as claimed in Claim 3, wherein each of m and n is 3.

5. A process as claimed in Claim 3 or 4, wherein each of $R^1$ and $R^2$ is a methyl radical.

6. A process as claimed in Claim 3 or 4, wherein each of $R^1$ and $R^2$ is a hydrogen atom.

7. A process as claimed in Claim 1 or 2, for producing dimethyl 5,5'-[3,6-pyridazinediylbis(thio)]bis[2,2-dimethylpentanoate]; dimethyl 5,5'-[(4-phenyl-3,6-pyridazinediyl)bis(thio)]-bis[2,2-dimethylpentanoate]; or dimethyl 5,5'-[(4-methyl-3,6-pyridazinediyl)bis(thio)]-bis[2,2-dimethylpentanoate]; or a pharmaceutically acceptable acid addition salt thereof.

8. A process as claimed in Claim 1 or 2, for producing dimethyl 6,6'-[3,6-pyridazinediylbis(thio)]bis[2,2-dimethylhexanoate] or a pharmaceutically acceptable acid addition salt thereof.

9. A process according to Claim 1 or 2 for producing dimethyl 8,8'-[3,6-pyridazinediylbis(thio)]bis[2,2-dimethyloctanoate) or a pharmaceutically acceptable acid addition salt thereof.

10. A process according to Claim 1 or 2 for producing 5,5'-[3,6-pyridazinediylbis(thio)]bis[2,2-dimethylpentanoic acid] or a pharmaceutically acceptable acid and/or base addition salt(s) thereof.

11. A process for producing a composition useful for treating arteriosclerosis in a mammal, which comprises combining a compound as produced by the process according to any one of Claims 1 to 10, or mixtures thereof, with a pharmaceutically acceptable carrier.

**Patentansprüche für die Vertragsstaaten: BE CH DE FR GB IT LI LU NL SE**

1. Eine Verbindung der folgenden Strukturformel

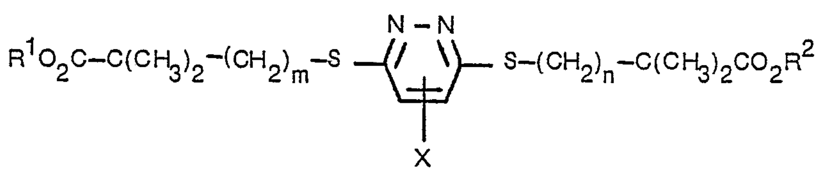

worin m und n, welche gleich oder verschieden sind, jeweils eine ganze Zahl von 3 bis 6 bedeuten; X für ein Wasserstoffatom, ein Phenylradikal oder ein Alkylradikal mit 1 bis 6 Kohlenstoffatomen steht; $R^1$ und $R^2$, welche gleich oder verschieden sind, jeweils ein Wasserstoffatom, ein Alkylradikal mit 1 bis 6 Kohlenstoffatomen oder ein pharmazeutisch akzeptables Kation repräsentieren; oder ein pharmazeutisch akzeptables Säureadditionssalz davon.

2. Eine Verbindung nach Anspruch 1, worin m und n gleich sind, und $R^1$ und $R^2$ gleich sind.

3. Eine Vebindung nach Anspruch 2, worin m und n jeweils 3 sind.

4. Eine Verbindung nach den Ansprüchen 2 oder 3, worin $R^1$ und $R^2$ jeweils ein Methylradikal darstellen.

5. Eine Verbindung nach den Ansprüchen 2 oder 3, worin $R^1$ und $R^2$ jeweils ein Wasserstoffatom repräsentieren.

6. Die Verbindung Dimethyl 5,5'-[3,6-pyridazindiyl-bis-(thio)]-bis-(2,2-dimethylpentanoat); Dimethyl-5,5'-[(4-phenyl-3,6-pyridazindiyl)-bis-(thio)]-bis-(2,2-dimethylpentanoat); oder Dimethyl-5,5'-[(4-methyl-

3,6-pyridazindiyl)-bis-(thio)]-bis-(2,2-dimethylpentanoat); oder ein pharmazeutisch akzeptables Säureadditionssalz davon.

7. Die Verbindung Dimethyl-6,6'-[3,6-pyridazindiyl-bis-(thio)]-bis-(2,2-dimethylhexanoat) oder ein pharmazeutisch akzeptables Säureadditionssalz davon.

8. Die Verbindung Dimethyl-8,8'-[3,6-pyridazindiyl-bis-(thio)]-bis-(2,2-dimethyloctanoat) oder ein pharmazeutisch akzeptables Säureadditionssalz davon.

9. Die Verbindung 5,5'-[3,6-Pyridazindiyl-bis-(thio)]-bis-(2,2-dimethylpentansäure) oder (ein) pharmazeutisch akzeptable(s) Säure- und/oder Basenadditionssalz(e) davon.

10. Verfahren zur Herstellung einer chemischen Verbindung nach Anspruch 1, dadurch gekennzeichnet, daß ein Pyridazin der folgenden Strukturformel

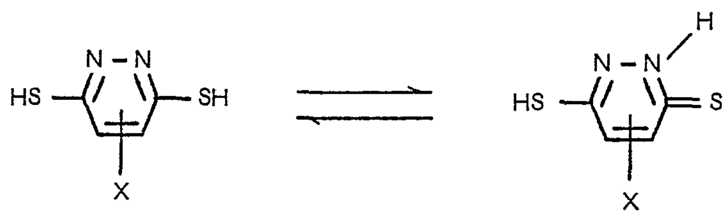

mit einem oder beiden der 2,2-Dimethylalkansäurederivate der folgenden Strukturformeln

$$Y^1—(CH_2)_m—C(CH_3)_2—CO_2R^1$$

$$Y^2—(CH_2)_n—C(CH_3)_2—CO_2R^2,$$

worin m, n, $R^1$ und X wie in Anspruch 1 definiert sind, und $Y^1$ und $Y^2$, welche gleich oder verschieden sind, jeweils ein Halogenatom darstellen, umgesetzt wird, wobei ausreichend 2,2-Dimethylalkansäurederivat(e) vorhanden ist (sind), um 2 Moleküle des Säurederivates mit einem des Pyridazins umzusetzen; und, wo es angebracht erscheint, das Reaktionsprodukt in ein pharmazeutisch akzeptables Säureadditionssalz übergeführt wird.

11. Verfahren zur Herstellung einer chemischen Verbindung nach Anspruch 1, dadurch gekennzeichnet, daß ein Pyridazin der folgenden Strukturformel

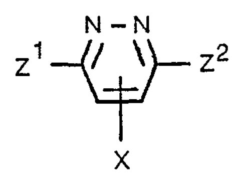

mit einem oder beiden der 2,2-Dimethylalkansäurederivate der folgenden Strukturformeln

$$HS(CH_2)_m—C(CH_3)_2—CO_2R^1$$

$$HS(CH_2)_n—C(CH_3)_2—CO_2R^2$$

worin m, n, $R^1$, $R^2$ und X wie in Anspruch 1 definiert sind, und $Z^1$ und $Z^2$, welche gleich oder verschieden sein können, jeweils ein Halogenatom darstellen, umgesetzt wird, wobei ausreichend 2,2-Dimethylalkansäurederivat(e) vorhanden ist (sind), um 2 Moleküle des Säurederivates mit einem Molekül des Pyridazins umzusetzen; und, wo es angebracht erscheint, das Reaktionsprodukt in ein pharmazeutisch akzeptables Säureadditionssalz übergeführt wird.

12. Eine zur Behandlung von Arteriosklerose von Säugetieren nützliche Zusammensetzung, dadurch gekennzeichnet, daß eine nach jeweils einem der Ansprüche 1 bis 9 hergestellte Verbindung oder eine Mischung davon mit einem pharmazeutisch akzeptablen Träger kombiniert ist.

**Patentansprüche für den Vertragsstaat: AT**

1..Verfahren zur Herstellung einer Verbindung der folgenden Strukturformel

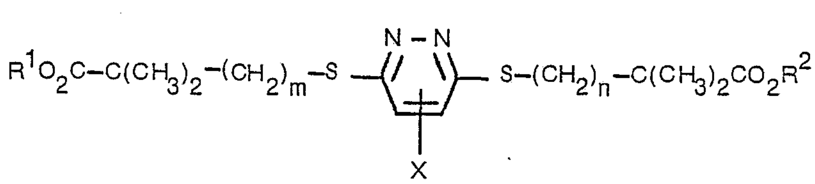

worin m und n, welche gleich oder verschieden sind, jeweils eine ganze Zahl von 3 bis 6 bedeuten; X für ein Wasserstoffatom, Phenylradikal oder Alkylradikal mit 1 bis 6 Kohlenstoffatomen steht; $R^1$ und $R^2$, welche gleich oder verschieden sind, jeweils ein Wasserstoffatom, ein Alkylradikal mit 1 bis 6 Kohlenstoffatomen oder ein pharmazeutisch akzeptables Kation repräsentieren; oder eines pharmazeutisch akzeptablen Säureadditionssalzes davon, dadurch gekennzeichnet, daß ein Pyridazin der folgenden Strukturformel II

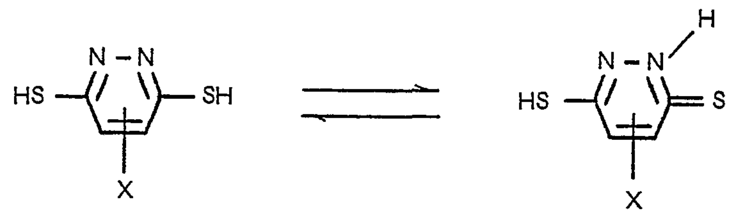

II

mit einem oder beiden der 2,2-Dimethylalkansäurederivate der folgenden Strukturformeln III

$$Y^1—(CH_2)_m—C(CH_3)_2—CO_2R^1$$
$$Y^2—(CH_2)_n—C(CH_3)_2—CO_2R^2 \quad III,$$

worin m, n, $R^1$ und X wie oben definiert sind, und $Y^1$ und $Y^2$, welche gleich oder verschieden sind, jeweils ein Halogenatom darstellen, umgesetzt wird, wobei ausreichend 2,2-Dimethylalkansäurederivat(e) vorhanden ist (sind), um 2 Moleküle des Säurederivates mit einem des Pyridazins umzusetzen; und, wo es angebracht erscheint, das Reaktionsprodukt in ein pharmazeutisch akzeptables Säureadditionssalz übergeführt wird.

2. Verfahren zur Herstellung einer Verbindung der folgenden Strukturformel

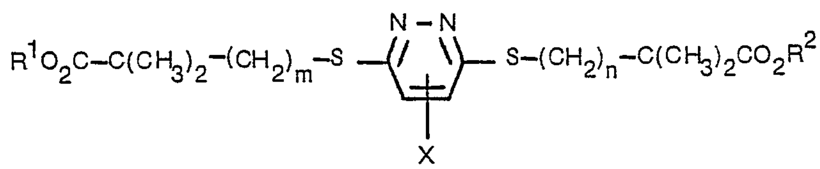

worin m und n, welche gleich oder verschieden sind, jeweils eine ganze Zahl von 3 bis 6 sind; X ein Wasserstoffatom, Phenylradikal oder Alkylradikal mit 1 bis 6 Kohlenstoffatomen bedeutet; $R^1$ und $R^2$, welche gleich oder verschieden sind, jeweils ein Wasserstoffatom, Alkylradikal mit 1 bis 6 Kohlenstoffatomen oder ein pharmazeutisch akzeptables Kation darstellen; oder eines pharmazeutisch akzeptablen Säureadditionssalzes davon, dadurch gekennzeichnet, daß ein Pyridazin der folgenden Strukturformel

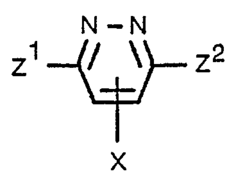

mit einem oder beiden der 2,2-Dimethylalkansäurederivate der folgenden Strukturformeln

$$HS(CH_2)_m—C(CH_3)_2—CO_2R^1$$
$$HS(CH_2)_n—C(CH_3)_2—CO_2R^2,$$

worin m, n, $R^1$, $R^2$ und X wie in Anspruch 1 definiert sind, und $Z^1$ und $Z^2$, welche gleich oder verschieden sein können, jeweils ein Halogenatom darstellen, wobei ausreichend 2,2-Dimethylalkansäurederivat(e) vorhanden ist (sind), um zwei Moleküle des Säurederivates mit einem Molekül des Pyridazins umzusetzen; und, wo es angebracht erscheint, das Reaktionsprodukt in ein pharmazeutisch akzeptables Säureadditionssalz übergeführt wird.

3. Verfahren nach den Ansprüchen 1 oder 2, wobei m und n gleich sind, und $R^1$ und $R^2$ gleich sind.

4. Verfahren nach Anspruch 3, wobei m und n jeweils 3 sind.

5. Verfahren nach den Ansprüchen 3 oder 4, wobei $R^1$ und $R^2$ jeweils ein Methylradikal sind.

6. Verfahren nach den Ansprüchen 3 oder 4, wobei $R^1$ und $R^2$ jeweils ein Wasserstoffatom darstellen.

7. Verfahren nach den Ansprüchen 1 oder 2 zur Herstellung von Dimethyl-5,5'-[3,6-pyridazindiyl-bis-(thio)] - bis - (2,2 - dimethylpentanoat); Dimethyl - 5,5' - [(4 - phenyl - 3,6 - pyridazindiyl) - bis(thio)] - bis -

(2,2 - dimethylpentanoat); oder Dimethyl - 5,5′ - [(4 - methyl - 3,6 - pyridazindiyl) - bis - (thio)] - bis - (2,2 - dimethylpentanoat); oder einem pharmazeutisch akzeptablen Säureadditionssalz davon.

8. Verfahren nach den Ansprüchen 1 oder 2 zur Herstellung von Dimethyl-6,6′-[3,6-pyridazindiyl-bis-(thio)]-bis-(2,2-dimethylhexanoat) oder einem pharmazeutisch akzeptablen Säureadditionssalz davon.

9. Verfahren nach Anspruch 1 oder 2 zur Herstellung von Dimethyl-8,8′-[3,6-pyridazindiyl-bis-(thio)]-bis-(2,2-dimethyloctanoat) oder einem pharmazeutisch akzeptablen Säureadditionssalz davon.

10. Verfahren nach den Ansprüchen 1 oder 2 zur Herstellung von 5,5′-[3,6-Pyridazindiyl-bis-(thio)]-bis-(2,2-dimethylpentansäure) oder (einem) pharmazeutisch akzeptablen Säure- und/oder Basenadditionssalz(en) davon.

11. Verfahren zur Herstellung einer bei der Behandlung von Arteriosklerose von Säugetieren nützlichen Zudammensetzung, dadurch gekennzeichnet, daß eine gemäß dem Verfahren nach jeweils einem der Ansprüche 1 bis 10 hergestellte Verbindung oder Mischungen davon mit einem pharmazeutisch akzeptablen Träger kombinert werden.

**Revendications pour les Etats contractants: BE CH DE FR GB IT LI LU NL SE**

1. Un composé ayant la formule structurelle suivante:

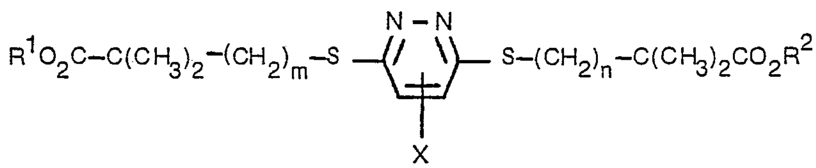

dans laquelle :

m et n, identiques ou différents, représent chacun un nombre entier de 3 à 6;

X représente un atome d'hydrogène, un radical phényl ou un radical alkyle possédant de 1 à 6 atomes de carbone;

$R^1$ et $R^2$, identiques ou différents, représentent chacun un atome d'hydrogène, un radical alkyle possédant de 1 à 6 atomes de carbone, ou un cation pharmaceutiquement acceptable ou un de leurs sels d'addition d'acides pharmaceutiquement acceptables.

2. Un composé selon la revendication 1, dans lequel m et n sont identiques et $R^1$ et $R^2$ sont identiques.

3. Un composé selon la revendication 2, dans lequel m et n sont chacun égaux à 3.

4. Un composé selon la revendication 2 ou 3, dans lequel $R^1$ et $R^2$ représentent chacun un radical méthyle.

5. Un composé selon la revendication 2 ou 3, dans lequel $R^1$ et $R^2$ représentent chacun un atome d'hydrogène.

6. Les composés diméthyl-5,5′-{3,6-pyridazinediylbis(thio)}bis(2,2-diméthyl-pentanoate); diméthyl-5,5′-{(4-phényl-3,6-pyridazinediyl)bis(thio)}bis(2,2-diméthyl-pentanoate); ou diméthyl-5,5′-{(4-méthyl-3,6-pyridazinediyl)bis(thio)}bis(2,2-diméthyl-pentanoate) ou leurs sels d'addition d'acides pharmaceutiquement acceptables.

7. Le composé diméthyl-6,6′-{3,6-pyridazinediylbis(thio)}bis(2,2-diméthyl-hexanoate) ou un de ses sels d'addition d'acides pharmaceutiquement acceptables.

8. Le composé diméthyl-8,8′-{3,6-pyridazinediylbis(thio)}bis(2,2-diméthyl-octanoate) ou un de ses sels d'addition d'acides pharmaceutiquement acceptables.

9. Le composé 5,5′-{3,6-pyridazinediylbis(thio)}bis(acide 2,2-diméthyl-pentanoïque) ou un de ses sels d'addition d'acides et/ou de bases pharmaceutiquement acceptables.

10. Procédé de préparation d'un composé chimique selon la revendication 1, consistant à faire réagir une pyridazine ayant la formule structurelle suivante:

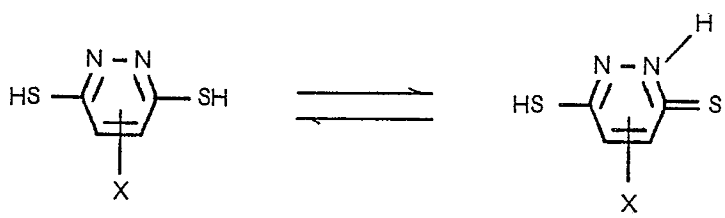

avec un ou deux des dérivés d'acide 2,2-diméthylalcanoïque ayant les formules structurelles suivantes:

$$Y^1\text{—}(CH_2)_m\text{—}C(CH_3)_2\text{—}CO_2R^1$$

$$Y^2\text{—}(CH_2)_n\text{—}C(CH_3)_2\text{—}CO_2R^2$$

dans lesquelles:

m, n, $R^1$ et X sont tels que définis dans la revendication 1; et

$Y^1$ et $Y^2$, identiques ou différents, représentent chacun un atome d'halogène,

et il suffit que, du (des) dérivé(s) de l'acide 2,2-diméthylalcanoïque, deux molécules du dérivé acide réagissent avec une seule molécule de pyridazine; et, lorsque cela est convenable à transformer le produit de la réaction en un sel d'addition d'acide pharmaceutiquement acceptable.

11. Procédé de préparation d'un composé chimique selon la revendication 1, consistant à faire réagir une pyridazine présentant la formule structurelle suivante:

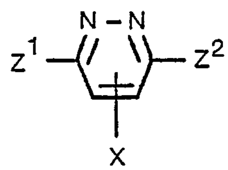

avec un ou deux des dérivés d'acide 2,2-diméthylalcanoïque présentant les formules structurelles suivantes:

$$HS(CH_2)_m—C(CH_3)_2—CO_2R^1$$

$$HS(CH_2)_n—C(CH_3)_2—CO_2R^2$$

dans lesquelles:

m, n, $R^1$ et X sont tels que définis dans la revendication 1; et $Z^1$ et $Z^2$, qui peuvent être identiques ou différents, représentent chacun un atome d'halogène,

et il suffit que, du (des) dérivé(s) de l'acide 2,2-diméthylalcanoïque, deux molécules du dérivé acide réagissent avec une seule molécule de la pyridazine; et lorsque cela est convenable, à transformer le produit de la réaction en un sel d'addition d'acide pharmaceutiquement acceptable.

12. Une composition utile pour le traitement de l'artériosclérose chez un mammifère, comprenant un ou plusieurs composés selon l'une quelconque des revendications 1 à 9, en combinaison avec un véhicule pharmaceutiquement acceptable.

**Revendications pour l'Etat contractant: AT**

1. Un procédé de préparation d'un composé ayant la formule structurelle suivante:

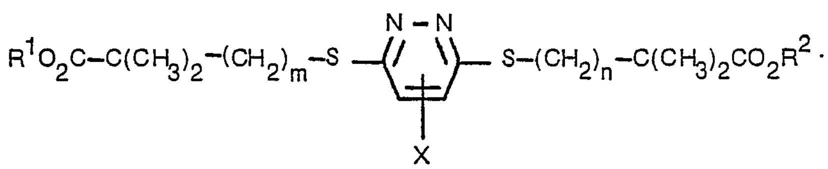

dans laquelle:

m et n, identiques ou différents, représentent chacun un nombre entier de 3 à 6;

X représente un atome d'hydrogène, un radical phényl ou un radical alkyle possédant de 1 à 6 atomes de carbone;

$R^1$ et $R^2$, identiques ou différents, représentent chacun un atome d'hydrogène, un radical alkyle possédant de 1 à 6 atomes de carbone, ou un cation pharmaceutiquement acceptable

ou un de ses sels d'addition d'acides pharmaceutiquement acceptables, ce procédé consistant à faire réagir une pyridazine ayant la formule structurelle suivante II.

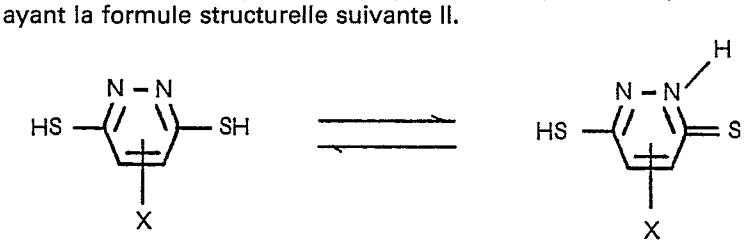

II

avec un ou deux des dérivés d'acide 2-2-diméthylalcanoïque ayant les formules structurelles suivantes III:

$$Y^1—(CH_2)_m—C(CH_3)_2—CO_2R^1$$

$$Y^2—(CH_2)_n—C(CH_3)_2—CO_2R^2$$

III

dans lesquelles:

m, n, $R^1$, $R^2$ ET X sont tels que définis dans la revendication 1; et

$Y^1$ et $Y^2$, identiques ou différents, représentent chacun un atome d'halogène,

et il suffit que, du (des) dérivé(s) de l'acide 2,2-diméthylalcanoïque, deux molécules du dérivé acid réagissent avec une seule molécule de la pyridazine; et, lorsque cela est convenable à transformer le produit de la réaction en un sel d'addition d'acide pharmaceutiquement acceptable.

2. Procédé de préparation d'un composé ayant la formule structurelle suivante:

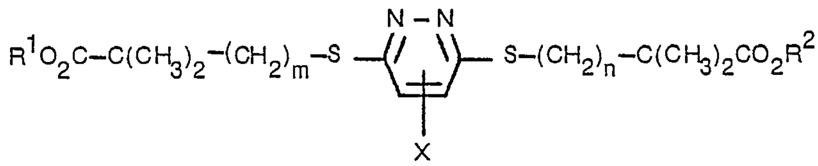

dans laquelle:

m et n, identiques ou différents, représentent chacun un nombre entier de 3 à 6;

X représente un atome d'hydrogène, un radical phényl ou un radical alkyle possédant de 1 à 6 atomes de carbone;

$R^1$ et $R^2$, identiques ou différents, représentent chacun un atome d'hydrogène, un radical alkyle possédant de 1 à 6 atomes de carbone, ou un cation pharmaceutiquement acceptable;

ou un de ses sels d'addition d'acides pharmaceutiquement acceptables, ce procédé consistant à faire réagir une pyridazine ayant la formule structurelle suivante:

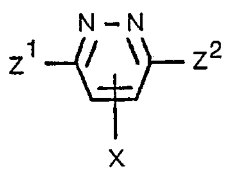

avec un ou deux des dérivés d'acide 2,2-diméthylalcanoïque ayant les formules structurelles suivantes:

$$HS(CH_2)_m—C(CH_3)_2—CO_2R^1$$

$$HS(CH_2)_n—C(CH_3)_2—CO_2R^2$$

dans lesquelles:

m, n, $R^1$, $R^2$ et X sont tels que définis dans la revendication 1; et

$Z^1$ et $Z^2$, identiques ou différents, représentent chacun un atome d'halogène,

et il suffit que, du (des) dérivé(s) de l'acide 2,2-diméthylalcanoïque, deux molécules du dérivé acide réagissent avec une seule molécule de la pyridazine; et lorsque cela est convenable, à transformer le produit de la réaction en un sel d'addition d'acide pharmaceutiquement acceptable.

3. Procédé selon la revendication 1 ou 2, dans lequel m et n sont identiques et $R^1$ et $R^2$ sont identiques.

4. Procédé selon la revendication 3, dans lequel m et n sont chacun égaux à 3.

5. Procédé selon la revendication 3 ou 4, dans lequel $R^1$ et $R^2$ représentent chacun un radical méthyle.

6. Procédé selon la revendication 3 ou 4, dans lequel $R^1$ et $R^2$ représentent chacun un atome d'hydrogène.

7. Procédé selon la revendication 1 ou 2, de préparation de diméthyl-5,5'-{3,6-pyridazinediylbis(thio)}bis(2,2 - diméthyl - pentanoate); diméthyl - 5,5' - {(4 - phényl - 3,6 - pyridazinediyl)bis(thio)}-bis(2,2 - diméthylpentanoate); ou diméthyl - 5,5' - {(4-méthyl - 3,6 - pyridazinediyl)bis(thio)}bis(2,2 - diméthyl - pentanoate; ou d'un de leurs sels d'addition d'acides pharmaceutiquement acceptables.

8. Procédé selon la revendication 1 ou 2, de préparation du diméthyl-6,6'-{3,6-pyridazinediylbis(thio)}-bis(2,2-diméthyl-hexanoate) ou un de ses sels d'addition d'acides pharmaceutiquement acceptables.

9. Procédé selon la revendication 1 ou 2, de préparation du diméthyl-8,8'-{3,6-pyridazinediylbis(thio)}bis(2,2-diméthyl-octanoate) ou un de ses sels d'addition d'acides pharmaceutiquement acceptables.

10. Procédé selon la revendication 1 ou 2, de préparation de 5,5'-{3,6-pyridazinediylbis(thio)}bis(acide 2,2-diméthyl-pentanoïque) ou un de ses sels d'addition d'acides et/ou de bases pharmaceutiquement acceptables.

11. Procédé de préparation d'une composition utile au traitement de l'artériosclérose chez un mammifère, consistant à combiner un ou plusieurs composés produit par le procédé selon l'une quelconque des revendications 1 à 10, avec un véhicule pharmaceutiquement acceptable.